# EUROPEAN PATENT SPECIFICATION

(11) **EP 4 079 281 B1**
(45) Date of publication and mention of the grant of the patent: **04.12.2024**
(21) Application number: 20902159.1
(22) Date of filing: 18.12.2020
(51) Int. Cl.: A61K 36/15, A61K 36/32, A61K 35/64, A61K 9/08, A61K 6/884, A61K 47/10, A61K 47/44, A61P 1/02, A61P 29/00, A61P 31/04, A61K 8/92, C08L 93/02, A61K 6/20, C08L 93/04, A61K 8/34, A61K 47/46

(54) **ANTI-INFLAMMATORY LIQUID COMPOSITION FOR COVERING ORAL MUCOSA, AND MEDICINAL COMPOSITION FOR PREVENTION AND/OR TREATMENT OF STOMATITIS USING SAME**
ENTZÜNDUNGSHEMMENDE FLÜSSIGE ZUSAMMENSETZUNG ZUM ABDECKEN DER MUNDSCHLEIMHAUT UND ARZNEIMITTEL ZUR VORBEUGUNG UND/ODER BEHANDLUNG VON STOMATITIS UNTER ANWENDUNG DERSELBEN
COMPOSITION LIQUIDE ANTI-INFLAMMATOIRE POUR RECOUVREMENT DE LA MUQUEUSE BUCCALE, ET COMPOSITION MÉDICINALE POUR LA PRÉVENTION ET/OU LE TRAITEMENT DE LA STOMATITE L'UTILISANT

(30) Priority: 19.12.2019 JP 2019229561
(43) Date of publication of application: 26.10.2022
(73) Proprietor: Hanic White Labo Co., Ltd., Tokyo 102-0082 (JP)
(72) Inventor: URAI, Kaoruko, Tokyo 102-0082 (JP); WADA, Sachiko, Chiba-shi, Chiba 260-0856 (JP); URAI, Yasutaka, Tokyo 102-0082 (JP)
(74) Representative: Winter, Brandl - Partnerschaft mbB
(86) International application number: PCT/JP2020/047388
(87) International publication number: WO 2021/125315

(56) References cited:
- EP-A1- 1 666 019
- WO-A1-2005/020937
- WO-A1-2009/124311
- JP-A- 2011 162 470
- JP-A- 2011 516 500
- JP-A- S62 142 112
- US-A1- 2009 142 282
- SUGIYAMA KENTARO ET AL: "Effects of the tooth-coating solution and its components on the proliferation and production of inflammatory cytokines in mitogen-activated human peripheral blood mononuclear cells", CURRENT TOPICS IN PHARMACOLOGY, 1 January 2019 (2019-01-01), XP093007456, Retrieved from the Internet <URL:http://www.researchtrends.net/tia/article_pdf.asp?in=0&vn=23&tid=11&aid=6410> [retrieved on 20221213]

## Description

### TECHNICAL FIELD

The present invention relates to a liquid composition for use in the treatment of inflammatory conditions of oral mucosa, which has an anti-inflammatory effect and an antibacterial effect, and suppresses or heals increased wound and inflammation in the oral cavity by applying the composition to the oral mucosa to form a film, as well as a liquid composition for use in the prevention and/or treatment of stomatitis.

### BACKGROUND ART

Conventionally, a liquid plaster, a liquid bandage, or the like has been proposed as a skin protectant for protecting a skin by forming a film on the skin in order to protect a wound and prevent irritation. Nitrocellulose, octyl cyanoacrylate, or the like is used as a film-forming agent for a liquid plaster, and ethyl acetate, ethyl ether, or the like is used as a solvent. Ethyl acetate and ethyl ether are toxic to the human body, and therefore it is not preferable to use such a liquid plaster in the oral cavity. In addition, although it is possible to protect external irritation by the film, the film-forming agent itself does not have an anti-inflammatory effect, and a medicinal ingredient having an anti-inflammatory effect is added.

Recently, as a therapeutic agent for inflammation in the oral cavity, a therapeutic patch for stomatitis has been proposed [Aftach (registered trademark), manufactured by TEIJIN PHARMA LIMITED. Sato Pharmaceutical Co., Ltd.: (see "Therapeutic Agent for Stomatitis/Aftach", New Drugs 2007 edition, published by Yakuji Nippo, Limited, pp. 285-286)]. In this oral therapeutic agent, a medicinal ingredient such as triamcinolone acetonide is mixed with a hydrophilic resin such as a carboxyvinyl polymer, and the mixture is formed into a patch, and the patch is applied to an affected area in order to aim at attaining a sustained release effect of the agent.

There has also been proposed an ointment agent for treating stomatitis which is in the form of an ointment and is applied to an affected area in the oral cavity [Ortexer (registered trademark) Oral Ointment 0.1%, manufactured by BEE BRAND MEDICO DENTAL. CO. LTD., sold by NIHON GENERIC Co., Ltd.: (see "Ortexer (registered trademark) Oral Ointment 0.1%" package insert, revised in December 2017 (2nd edition))]. This ointment is obtained by adding triamcinolone acetonide, which is an anti-inflammatory steroid drug, to a gelling base.

Furthermore, oral care compositions focusing on the antibacterial activity of rosin have also been proposed (see JP H04-66523 A and JP 2009-514789 A). In addition, conventionally, as an oral retention type base which forms an adhesive film by saliva when applied to the oral cavity and reliably remains in an affected area for a long time without being flowed by saliva, there has been disclosed an invention related to an oral retention type base capable of forming an adhesive film, which is formed in a liquid or paste form by dissolving a film-forming polymeric substance soluble in a lower alcohol and insoluble or poorly soluble in water (such as ethyl cellulose or polyvinyl acetate) and a tackifier resin (such as a rosin resin or a shellac resin) in an alcohol solvent (see JP S62-142112 A).

EP 1 666 019 A1 discloses an anti-caries tooth coating composition comprising shellac, rosin and solvent.

WO 2009/124311 A1 discloses an anti-caries tooth varnish composition comprising shellac, colophonium and ethanol.

Non-Patent Document (Sugiyama Kentaro ET AL: "Effects of the tooth-coating solution and its components on the proliferation and production of inflammatory cytokines in mitogen-activated human peripheral blood mononuclear cells", Current topics in Pharmacology, 1 January 2019 (2019-01-01), XP093007456) discloses a composition used for tooth coating, comprising rosin, copal and shellac.

US 2009/142282 A1 discloses a varnish composition for the treatment of dental caries comprising rosin and ethanol as solvent.

### SUMMARY OF INVENTION

### Technical Problem

The therapeutic patch for stomatitis (Aftach) used in the oral cavity contains triamcinolone acetonide as a main ingredient, and is a circular solid with a diameter of about 7 mm and a thickness of 1.1 mm. When the patch of this circular solid is applied to the affected area, the patch is pressed with a finger to allow the patch to be attached to the area. Not only the attached patch causes pain and suffering, but also the patch is immediately removed when touched with a tongue because the patch is thick. In addition, the touch on the tongue is poor and causes a considerable discomfort, and there is a disadvantage that, for example, the patch is peeled off immediately after eating food or drinking water, which is not suitable for children. In hospitalized patients, elderly persons, and the like, there is a high possibility that the patch is accidentally swallowed, and there is a possibility that the patch adheres to the esophageal mucosa to cause serious complications.

In addition, the above-described ointment agent for treating stomatitis (Ortexer) used in the oral cavity contains triamcinolone acetonide as a main ingredient, and a finger or a cotton swab is used to apply this ointment to an affected area, but it is difficult to allow the ointment to adhere to the area. Thus, the agent is not particularly suitable for children. Furthermore, the attached ointment evokes a sticky and rough feeling and an uncomfortable feeling, and also a feeling of discomfort in taste. Furthermore, since the ointment is removed when a tongue or the like touches it, the duration time is also short. In addition, there is a disadvantage that it is necessary to avoid food and drink for a while after the application of the ointment.

In the periodontal pathogen growth inhibitor described in JP H04-66523 A, a rosin, a processed rosin, or the like as an inhibitor against oral bacteria involved in the development of periodontal disease or gingivitis is added to an oral composition such as a chewing gum, a candy, a toothpaste, or a mouth rinse. Note that the addition amount thereof is as very small as 0.001 to 1 wt%. The main ingredients of the chewing gum described in JP H04-66523 A are a gum base and saccharides, and the chewing gum remains in the oral cavity for a long time, but does not have an adherence property enough for the chewing gum to adhere to the mucous membrane in the oral cavity. Rather, materials that do not adhere to the teeth or mucous membrane in the oral cavity are selected and used. As described above, the addition amount of the rosin and the processed rosin is very small, and thus the rosin and the processed rosin are hardly involved in the adherence property of the product. The main ingredients of the candy described in JP H04-66523 A are granulated sugar and starch syrup, and thus materials that do not adhere to the teeth or mucous membrane in the oral cavity are selected. The addition amount of the rosin and the processed rosin is very small, and thus the rosin and the processed rosin are hardly involved in the adherence property of the product. The main ingredients of the toothpaste described in JP H04-66523 A are polishing agents such as dibasic calcium phosphate and glycerin, and materials that do not adhere to the teeth or mucous membrane in the oral cavity are selected as materials of the product. The mouth rinse described in JP H04-66523 A contains water and an ethanol solution as main ingredients, and only trace amounts of the rosin and the processed rosin are added thereto. Therefore, although this mouth rinse is a liquid composition, it does not have the ability to form a film on the mucous membrane in the oral cavity.

In the oral care composition described in JP 2009-514789 A, there is used a composition containing a tree resin or an extract thereof or a derivative thereof as an antimicrobial agent against bacteria involved in caries, gingivitis, and other periodontal diseases. As the tree resin, a rosin or a colophonium is used. Under real conditions of the use of the resin, this antimicrobial agent is added to a chewing gum, a dentifrice, or a mouthwash in a very small amount. The amount of the agent to be added is limited to a range of 1 to 10,000 ppm (i.e., a range of 0.0001 to 1%) in the claims. In Examples, the amount of the agent to be added to the mouth rinse is in a range of 0.01 to 0.001%, and the amount of the agent to be added to the toothpaste is in a range of 0.1 to 0.001%, and each of the amounts is very small. In addition, since the mouthwash as a product of the Examples is prepared by adding large amounts of ethanol and water to a 0.1% aqueous alkali solution of rosin acid, the mouthwash has no ability to adhere to the mucous membrane in the oral cavity to form a film. Furthermore, the toothpaste is also produced by adding polishing agents such as silica and titanium dioxide, and thickeners such as glycerin and xanthan gum. However, the amount of the rosin is as small as 0.001%, and the rosin does not function at all as an adhesive material or a film-forming material. In addition, the materials are selected so that the dentifrice product itself does not adhere to the mucous membrane in the oral cavity.

Furthermore, according to the study of the present inventors, it has been found that the retention type base capable of forming an oral film described in JP S62-142112 A has a problem in terms of quick-drying property. It is presumed that this is because the content of the film-forming polymeric substance such as ethyl cellulose or polyvinyl acetate is relatively large.

An object of the present invention is to provide an anti-inflammatory liquid composition for use in the treatment of inflammatory conditions of oral mucosa, which is different from the conventional oral care products as described above, adheres to a mucous membrane in the oral cavity to form a film excellent in quick-drying property and durability, covers, for example, an affected area where a wound or inflammation is developed to protect from external irritation, and prevents or heals increased wound and inflammation.

### Solution to Problem

The present inventors have carried out a diligent study to solve the problem described above. As a result, the present inventors have found that the above problem can be solved by using, as an anti-inflammatory liquid composition for use in the treatment of inflammatory conditions of oral mucosa, a composition containing a rosin and a shellac (and in some cases, a copal) in a predetermined content in a solvent, and have completed the present invention.

That is, the present invention is defined in the subject-matter of claim 1 and claim 9. Preferred embodiments of the present invention are the subject-matter of the dependent claims

### Advantageous Effects of Invention

According to the present invention, there is provided an anti-inflammatory liquid composition for the use in the treatment of inflammatory conditions of oral mucosa which is different from conventional oral care products, and with which the above-described problem can be solved.

### DESCRIPTION OF EMBODIMENTS

One aspect of the present invention is an anti-inflammatory liquid composition for use in the treatment of inflammatory conditions of oral mucosa including: a rosin; a shellac; and a solvent in which a content of the rosin is 1 wt% or more and 15 wt% or less relative to a total amount of the composition, and a content of the shellac is 35 wt% or more and 45 wt% or less relative to the total amount of the composition, and a total content of the rosin, the shellac, and a copal is 45 wt% or more and 55 wt% or less relative to the total amount of the composition.

It is known that the rosin has antibacterial and anti-inflammatory effects against cariogenic bacteria and periodontopathic bacteria, as described in the documents in the related art. However, it has not been known that the wound site or inflamed site is covered with a rosin-containing film in order to protect the inflammation, wound, or the like in the oral cavity from external irritation and at the same time to maintain antibacterial and anti-inflammatory effects. As a result of studies by the present inventors, it has been demonstrated that the anti-inflammatory effect of the rosin is exhibited when the content of the rosin is more than 0.1 wt%. Therefore, in the case of using the composition according to the present invention in the oral cavity, the lower limit value of the content of the rosin is set to 1 wt% in consideration of gradual dissolution and permeation, on the assumption that the above-mentioned effect is exhibited when the content of the rosin is 10 times that of the composition.

In addition, since the rosin is a resin ingredient obtained from pine tar, an ethanol solution of rosin has a film-forming property. However, when the film is formed with a solution of rosin alone, there is a problem that the durability of the film is short. Also, in this case, there is a problem that irritation to the mucous membrane is strong, there is a stiff feeling due to the film in the oral cavity, and there is discomfort at the time of use. Furthermore, generally, rosin is commercially available as solid gum rosin. When the rosin is used in the composition for use in the treatment of inflammatory conditions of oral mucosa, it is necessary that the rosin is dissolved in a solvent such as ethanol and used. Here, in order to produce an ethanol solution of rosin, the rosin is put into ethanol and dissolved under stirring. Although it is sufficient to stir the rosin solution for several minutes when the concentration of the rosin solution is several wt%, it may take several hours to stir the solution when the concentration increases. In addition, since the rosin is irritating, when the amount of rosin is increased, the affected area may be stimulated to cause pain, and therefore it is not preferable to excessively increase the concentration of the rosin. Furthermore, when the concentration of the rosin dissolved in the ethanol solvent is excessively increased, there are problems that the surface of the film formed by applying the rosin to the affected area is rough, the touch on the tongue is deteriorated, and not only discomfort is given, but also the surface of the film is cracked and easily peeled off.

On the other hand, since the shellac as a vehicle (base material) for film formation has better performance than the rosin, it is considered that the shellac is further added in addition to the rosin, resulting in more preferable blending of the base material. Usually, shellac is commercially available as a 20 to 50% ethanol solution. Therefore, the addition of ethanol to the commercially available solution enables a shellac solution having a concentration lower than the commercially available solution to be easily obtained. In order to obtain an ethanol solution having a concentration higher than 50%, preparation can be performed by evaporating the ethanol portion in a warm bath at around 40°C or by adding dry powder shellac to the commercially available solution.

The anti-inflammatory liquid composition for use in the treatment of inflammatory conditions of oral mucosa according to the present invention which has been completed based on the above findings is characterized in that the composition contains resin ingredients: a rosin and a shellac, and solvents thereof as main ingredients. Ethanol is preferably used as the solvent.

In the anti-inflammatory liquid composition for use in the treatment of inflammatory conditions of oral mucosa according to the present invention, the content of the rosin as the resin ingredient described above is 1 wt% or more and 15 wt% or less, and preferably 1 wt% or more and 10 wt% or less relative to the total amount of the composition. Similarly, the content of the shellac as the resin ingredient is 35 wt% or more and 45 wt% or less, and preferably 35 wt% or more and 40 wt% or less relative to the total amount of the composition. It is essential that the total content of the rosin, the shellac, and a copal is 45 wt% or more and 55 wt% or less, and the total content is preferably 47 wt% or more and 51 wt% or less relative to the total amount of the composition. Here, the composition according to the present invention does not necessarily contain the copal, but when the composition does not contain the copal, the total content of the rosin and the shellac satisfies the above definition. When the anti-inflammatory liquid composition for use in the treatment of inflammatory conditions of oral mucosa according to the present invention having such characteristics is applied to a mucous membrane in the oral cavity, a solvent such as ethanol evaporates and a resin film is formed. The content of the solvent in the anti-inflammatory liquid composition for use in the treatment of inflammatory conditions of oral mucosa according to the present invention is 55 wt% or less, and preferably 53 wt% or less. On the other hand, the lower limit of the content of the solvent is preferably 40 wt% or more, more preferably 44 wt% or more, and particularly preferably 49 wt% or more. For example, when the solvent is ethanol, the content of ethanol in the composition is preferably 53 wt% or less. On the other hand, the lower limit of the content of ethanol is preferably 40 wt% or more, more preferably 44 wt% or more, and particularly preferably 49 wt% or more. The content of the solvent (e.g., ethanol) is preferably in a range of 49 to 53 wt%.

Furthermore, in the anti-inflammatory liquid composition for use in the treatment of inflammatory conditions of oral mucosa according to the present invention, the contents of ethyl cellulose, polyvinyl acetate, and cellulose nitrate (the total content when a plurality of these materials is contained) are preferably less than 0.5 wt%. More preferably, the contents of film-forming polymeric substances including ethyl cellulose, polyvinyl acetate, and cellulose nitrate (excluding the rosin, the shellac, and the copal) are less than 0.5 wt%. This is because when the contents of the ingredients described above are 0.5 wt% or more, the quick-drying property of the film formed in the oral cavity may be deteriorated. At the time of application of the composition according to the present invention, it is necessary to keep the mouth open until the composition dries, whereby the deterioration of the quick-drying property increases the pain of the user. The content of each of the ingredients described above is preferably 0.45 wt% or less, more preferably 0.3 wt% or less, still more preferably 0.2 wt% or less, particularly preferably 0.1 wt% or less, and most preferably 0 wt% (free). The term "film-forming polymeric substance" means a polymer capable of forming a thin film on the surface of an object in a drying step.

It is preferable that the anti-inflammatory liquid composition for use in the treatment of inflammatory conditions of oral mucosa according to the present invention further contains a copal as a resin ingredient. According to the composition according to the present invention, the surface of the film may be rough due to the blending of the rosin, but there is an advantage that the surface of the film formed can be made smoother by further containing the copal, and the stiffness can be reduced. The content of the copal is not particularly limited. Since the copal has a unique resin odor, when the blending amount of the copal is too large, the stiffness may be conversely increased. Thus, the content of the copal is preferably more than 0 wt% and 10 wt% or less, and more preferably 0.1 wt% or more and 8 wt% or less relative to the total amount of the composition.

The anti-inflammatory liquid composition for use in the treatment of inflammatory conditions of oral mucosa according to the present invention may further contain an accessory ingredient as long as the effect of the present invention is not adversely affected. Examples of the accessory ingredient include a medicinal ingredient and a moisturizing ingredient. The contents of these accessory ingredients are preferably 1 wt% or less relative to the total amount of the composition.

Examples of the medicinal ingredient that can be contained as an accessory ingredient include one or two or more selected from the group consisting of, for example, anti-inflammatory agents such as azulene sulfonate sodium and glycyrrhizic acid; antihistamines such as diphenhydramine hydrochloride and chlorpheniramine maleate; anti-inflammatory analgesics such as triamcinolone acetonide and dexamethasone acetate; antibiotics such as cefaclor, amoxicillin, erythromycin, and kanamycin; vitamin preparations such as vitamin B1 and vitamin E; and herbal medicines or Kampo preparations such as Angelica root, cinnamon bark, turmeric, and licorice.

Examples of the moisturizing ingredient that can be contained as the accessory ingredient include one or two or more selected from the group consisting of glycerin, wheat germ oil, macadamia nut oil, olive oil, jojoba oil, castor oil, and a plant extract. Furthermore, a titanium-containing ingredient such as titanium oxide or titanium dioxide coated mica may be added as an accessory ingredient.

The anti-inflammatory liquid composition for use in the treatment of inflammatory conditions of oral mucosa according to the present invention contains resin ingredients: a rosin and a shellac and solvents thereof as main ingredients. Thus, when the composition is applied to an inflamed area on the mucous membrane in the oral cavity using a brush or the like, a film can be formed to cover the inflamed site within about 20 seconds. Accordingly, it is possible to prevent external irritation. As a result, the pain of the affected area can be alleviated and the patient's burden can be reduced. In addition, since the rosin contained in the composition according to the present invention contains an anti-inflammatory ingredient, it is possible to prevent the expansion of the inflamed site. Furthermore, since the film of the composition according to the present invention covers and protects the affected area, healing of the inflamed site can be promoted.

It is also found that the rosin suppresses T cell mitogen (concanavalin A)-responsive proliferation of human peripheral-blood mononuclear cells (PBMCs), and inhibits production of five cytokines (proteins secreted from cells of the immune system: IL-6, IL-10, TNF-α, IFN-γ, and IL-17) from PBMCs in a concentration-dependent manner.

Since the composition according to the present invention contains an ingredient used as a food additive as a main ingredient, the composition is safe to be swallowed. In addition, even in the case where the film is swallowed by mistake, the film dissolves naturally and does not adhere to the mucous membrane of esophagus or the like, so there is no possibility of complication occurrence, or the like.

It is essential that the content of the rosin as the resin ingredient described above is 1 wt% or more relative to the total amount of the composition. This is in consideration of the fact that although the antimicrobial effect of the rosin is recognized from 0.1 wt%, the rosin is gradually dissolved and permeated to exert its effect. On the other hand, the upper limit of the content of the rosin is 15 wt% or less relative to the total amount of the composition. When the content of the rosin is more than 15 wt%, the surface of the formed film becomes stiff and the touch on the tongue is deteriorated, or cracking occurs and the film is easily peeled off. This is problematic. In addition, from the viewpoint of production, in the case of preparing the composition according to the present invention by dissolving the rosin in an ethanol solution (usually, a solution having a concentration of 50%) of shellac, when the content of the rosin exceeds 15 wt%, it takes one day or more to dissolve the rosin, leading to deterioration of productivity. This is problematic.

It is essential that the total content of the rosin and the shellac (and further a copal in the case of containing the copal) as resin ingredients is 45 wt% or more and 55 wt% or less relative to the total amount of the composition. Here, when the total content is less than 45 wt%, there is a problem that the adhesion of the formed film to the mucous membrane and the durability are deteriorated and the film is easily peeled off. In addition, as a result of the relative increase in the content of the solvent, the irritation to the mucous membrane may be enhanced. On the other hand, when the total content is more than 55 wt%, there is also a problem that the surface of the film becomes hard to cause increased stiffness, and the quick-drying property is deteriorated. When the total content is 55 wt% or less, the composition according to the present invention is dried in about 10 to 20 seconds, but this degree is the practical limit. When the total content exceeds 55 wt%, the drying time becomes long, and when the total content is, for example, 60 wt%, it takes several minutes to dry the composition. At the time of application of the composition according to the present invention, it is necessary to keep the mouth open until the composition dries, whereby the deterioration of the quick-drying property increases the pain of the user.

As described above, it is essential that the content of the rosin in the composition according to the present invention is 1 wt% or more and 15 wt% or less relative to the total amount of the composition. The content of the shellac in the composition according to the present invention was set to a range of 35 wt% or more and 45 wt% or less relative to the total amount of the composition. As a result, the feeling of the surface of the film is made smooth, and it is possible to form a film having elasticity and high durability. Note that the film formed by applying the composition according to the present invention is practically tasteless, and even when a tongue or the like touches the film, the film cannot be easily taken off, and there is no need to refrain from drinking or eating after application.

As described above, when the composition according to the present invention further contains a copal, the total content of the rosin, the shellac, and the copal is 45 wt% or more and 55 wt% or less. With such a configuration, the content of ethanol or the like as a solvent is 55 wt% or less, and the irritation can be reduced. Note that the film formed by the composition containing the rosin, the shellac, and the copal is not easily peeled off even when the film comes into contact with water because the film is hardly soluble in water, and there is an advantage that the film has durability for several hours even when the film is applied to a portion with significant movement in the oral cavity. In addition, when the film is applied to gums or the like with little movement, it is possible to form a film having durability for 6 hours or more.

The composition according to the present invention not only has an effect of protecting stomatitis by coating the stomatitis to suppress inflammation, but also has an effect of suppressing proliferation of oral bacteria such as cariogenic bacteria and periodontopathic bacteria by the antibacterial activity of the rosin gradually dissolved in saliva because the film remains in the oral cavity for a long time. Alternatively, the composition is also available as a sustained oral dosage form or a new dosage form of transdermal absorption. Hence, according to another aspect of the present invention, there is also provided a pharmaceutical composition for preventing and/or treating stomatitis, including the anti-inflammatory liquid composition for use in the treatment of inflammatory conditions of oral mucosa according to the present invention.

### EXAMPLES

The effects of the present invention are described with reference to the following Examples and Comparative Examples.

### [Preparation Example of Liquid Composition]

Liquid compositions of Examples and Comparative Examples were prepared by the following method. The blending amounts of raw materials in each of the Examples and each of the Comparative Examples are shown in Table 1 below. In addition, the blending amounts of components in each of the Examples and each of the Comparative Examples are shown in Table 2 below. In principle, the Examples and Comparative Examples shown in Tables 1 and 2 are arranged in ascending order of resin concentration.

### (Example 1)

10.0 wt% of gum rosin (LAWTER ARGENTINA S.A.), 80.0 wt% of Laccoat 50EDS (50% shellac-ethanol solution) (manufactured by THE JAPAN SHELLAC INDUSTRIES, LTD.), 3.3 wt% of Copal HJ-01 (30% copal-ethanol solution) (manufactured by Gifu Shellac Manufacturing Co., Ltd.), and 6.7 wt% of ethanol (99.5) Imazu Class 1 (absolute ethanol) (manufactured by IMAZU CHEMICAL CO. LTD.) were well mixed and dissolved with a stirrer to obtain a composition in the form of uniform solution.

### (Example 2)

8.0 wt% of gum rosin, 80.0 wt% of Laccoat 50EDS, 6.7 wt% of Copal HJ-01, and 5.3 wt% of Imazu Class 1 ethanol (99.5) were well mixed and dissolved with a stirrer to obtain a composition in the form of uniform solution.

### (Example 3)

6.0 wt% of gum rosin, 80.0 wt% of Laccoat 50EDS, 13.3 wt% of Copal HJ-01, and 0.7 wt% of Imazu Class 1 ethanol (99.5) were well mixed and dissolved with a stirrer to obtain a composition in the form of uniform solution.

### (Example 4)

10.0 wt% of gum rosin, 80.0 wt% of Laccoat 50EDS, and 10.0 wt% of ethanol (99.5) Imazu Class 1 were well mixed and dissolved with a stirrer to obtain a composition in the form of uniform solution.

### (Example 5)

10.0 wt% of gum rosin, 70.0 wt% of Laccoat 50EDS, 16.7 wt% of Copal HJ-01, and 3.3 wt% of Imazu Class 1 ethanol (99.5) were well mixed and dissolved with a stirrer to obtain a composition in the form of uniform solution.

### (Example 6)

10.0 wt% of gum rosin, 76.0 wt% of Laccoat 50EDS, 3.3 wt% of Copal HJ-01, and 10.7 wt% of Imazu Class 1 ethanol (99.5) were well mixed and dissolved with a stirrer to obtain a composition in the form of uniform solution.

### (Example 7)

1.0 wt% of gum rosin, 80.0 wt% of Laccoat 50EDS, 15.0 wt% of a solution obtained by concentrating Copal HJ-01 to a 40% ethanol solution, and 4.0 wt% of Imazu Class 1 ethanol (99.5) were well mixed and dissolved with a stirrer to obtain a composition in the form of uniform solution.

### (Example 8)

3.0 wt% of gum rosin, 80.0 wt% of Laccoat 50EDS, 10.0 wt% of a solution obtained by concentrating Copal HJ-01 to a 40% ethanol solution, and 7.0 wt% of Imazu Class 1 ethanol (99.5) were well mixed and dissolved with a stirrer to obtain a composition in the form of uniform solution.

### (Example 9)

6.0 wt% of gum rosin, 80.0 wt% of Laccoat 50EDS, 2.5 wt% of a solution obtained by concentrating Copal HJ-01 to a 40% ethanol solution, and 11.5 wt% of Imazu Class 1 ethanol (99.5) were well mixed and dissolved with a stirrer to obtain a composition in the form of uniform solution.

### (Comparative Example 1)

33.3 wt% of gum rosin and 66.7 wt% of Laccoat 50EDS were well mixed and dissolved with a stirrer to obtain a composition in the form of uniform solution.

### (Comparative Example 2)

30.0 wt% of gum rosin, 60.0 wt% of Laccoat 50EDS, and 10.0 wt% of ethanol (99.5) Imazu Class 1 were well mixed and dissolved with a stirrer to obtain a composition in the form of uniform solution.

### (Comparative Example 3)

10.0 wt% of gum rosin, 71.4 wt% of a solution obtained by concentrating Laccoat 50EDS to a 70% ethanol solution, and 18.6 wt% of Imazu Class 1 ethanol (99.5) were well mixed and dissolved with a stirrer to obtain a composition in the form of uniform solution.

### (Comparative Example 4)

33.3 wt% of gum rosin and 66.7 wt% of Copal HJ-01 were well mixed and dissolved with a stirrer to obtain a composition in the form of uniform solution.

### (Comparative Example 5)

25.0 wt% of gum rosin, 50.0 wt% of Laccoat 50EDS, and 25.0 wt% of ethanol (99.5) Imazu Class 1 were well mixed and dissolved with a stirrer to obtain a composition in the form of uniform solution.

### (Comparative Example 6)

10.0 wt% of gum rosin, 50.0 wt% of Laccoat 50EDS, and 40.0 wt% of Copal HJ-01 were well mixed and dissolved with a stirrer to obtain a composition in the form of uniform solution.

### (Comparative Example 7)

17.0 wt% of gum rosin, 80.0 wt% of Laccoat 50EDS, and 3.0 wt% of ethanol (99.5) Imazu Class 1 were well mixed and dissolved with a stirrer to obtain a composition in the form of uniform solution.

### (Comparative Example 8)

8.0 wt% of gum rosin, 64.0 wt% of Laccoat 50EDS, 1.7 wt% of Copal HJ-01, and 26.3 wt% of Imazu Class 1 ethanol (99.5) were well mixed and dissolved with a stirrer to obtain a composition in the form of uniform solution.

### (Comparative Example 9)

10.0 wt% of gum rosin, 60.0 wt% of Laccoat 50EDS, and 30.0 wt% of ethanol (99.5) Imazu Class 1 were well mixed and dissolved with a stirrer to obtain a composition in the form of uniform solution.

### (Comparative Example 10)

10.0 wt% of gum rosin, 50.0 wt% of Laccoat 50EDS, and 40.0 wt% of ethanol (99.5) Imazu Class 1 were well mixed and dissolved with a stirrer to obtain a composition in the form of uniform solution.

### (Comparative Example 11)

5.0 wt% of gum rosin, 30.0 wt% of Laccoat 50EDS, and 65.0 wt% of ethanol (99.5) Imazu Class 1 were well mixed and dissolved with a stirrer to obtain a composition in the form of uniform solution.

### (Comparative Example 12)

50.0 wt% of gum rosin and 50.0 wt% of ethanol (99.5) Imazu Class 1 were well mixed and dissolved with a stirrer to obtain a composition in the form of uniform solution.

**[Table 1]**

| Table 1 Formulation of raw material of anti-inflammatory liquid composition for use in the treatment of inflammatory conditions of oral mucosa | | | | | |
|---|---|---|---|---|---|
| Sample Nos. | Formulation ratios of raw materials (wt%) | | | | |
| | Gum rosin | Laccoat *1 | Copal solution *2 | Ethanol *3 | Total |
| Example 1 | 10.0 | 80.0 | 3.3 | 6.7 | 100.0 |
| Example 2 | 8.0 | 80.0 | 6.7 | 5.3 | 100.0 |
| Example 3 | 6. 0 | 80.0 | 13.3 | 0.7 | 100.0 |
| Example 4 | 10.0 | 80.0 | 0. 0 | 10.0 | 100.0 |
| Example 5 | 10.0 | 70.0 | 16.7 | 3.3 | 100.0 |
| Example 6 | 10.0 | 76.0 | 3.3 | 10.7 | 100.0 |
| Example 7 | 1.0 | 80.0 | 15.0 *4 | 4. 0 | 100.0 |
| Example 8 | 3. 0 | 80.0 | 10.0 *4 | 7.0 | 100.0 |
| Example 9 | 6. 0 | 80.0 | 2.5 *4 | 11.5 | 100.0 |
| Comparative Example 1 | 33.3 | 66.7 | 0.0 | 0 . 0 | 100.0 |
| Comparative Example 2 | 30.0 | 60.0 | 0.0 | 10.0 | 100.0 |
| Comparative Example 3 | 10.0 | 71.4 *5 | 0.0 | 18.6 | 100.0 |
| Comparative Example 4 | 33.3 | 0.0 | 66.7 | 0.0 | 100.0 |
| Comparative Example 5 | 25.0 | 50.0 | 0.0 | 25.0 | 100.0 |
| Comparative Example 6 | 10.0 | 50.0 | 40.0 | 0.0 | 100.0 |
| Comparative Example 7 | 17.0 | 80.0 | 0.0 | 3.0 | 100.0 |
| Comparative Example 8 | 8.0 | 64.0 | 1.7 | 26.3 | 100.0 |
| Comparative Example 9 | 10.0 | 60.0 | 0.0 | 30.0 | 100.0 |
| Comparative Example 10 | 10.0 | 50.0 | 0.0 | 40.0 | 100.0 |
| Comparative Example 11 | 5.0 | 30.0 | 0.0 | 65.0 | 100.0 |
| Comparative Example 12 | 50.0 | 0.0 | 0.0 | 50.0 | 100.0 |

| | | | | | |
|---|---|---|---|---|---|
| *1 = Laccoat 50EDS *2 = Copal HJ-01 *3 = Ethanol (99.5) Imazu Class 1 *4 = Solution obtained by concentrating Copal HJ-01 to a 40% copal-ethanol solution *5 = Solution obtained by concentrating Laccoat 50EDS to a 70% shellac-ethanol solution | | | | | |

**[Table 2]**

| Table 2 Formulation of raw material of anti-inflammatory liquid composition for use in the treatment of inflammatory conditions of oral mucosa | | | | | |
|---|---|---|---|---|---|
| Sample Nos. | Formulation ratios of raw materials (wt%) | | | | |
| | Rosin | Shellac | Copal | Ethanol | Total |
| Example 1 | 10.0 | 40.0 | 1.0 | 49.0 | 100.0 |
| Example 2 | 8.0 | 40.0 | 2.0 | 50.0 | 100.0 |
| Example 3 | 6.0 | 40.0 | 4.0 | 50.0 | 100.0 |
| Example 4 | 10.0 | 40.0 | 0.0 | 50.0 | 100.0 |
| Example 5 | 10.0 | 35.0 | 5.0 | 50.0 | 100.0 |
| Example 6 | 10.0 | 38.0 | 1.0 | 51.0 | 100.0 |
| Example 7 | 1.0 | 40.0 | 6.0 | 53.0 | 100.0 |
| Example 8 | 3.0 | 40.0 | 4.0 | 53.0 | 100.0 |
| Example 9 | 6.0 | 40.0 | 1.0 | 53.0 | 100.0 |
| Comparative Example 1 | 33.3 | 33.3 | 0.0 | 33.4 | 100.0 |
| Comparative Example 2 | 30.0 | 30.0 | 0.0 | 40.0 | 100.0 |
| Comparative Example 3 | 10.0 | 50.0 | 0.0 | 40.0 | 100.0 |
| Comparative Example 4 | 33.3 | 0. 0 | 20.0 | 46.7 | 100.0 |
| Comparative Example 5 | 25.0 | 25.0 | 0.0 | 50.0 | 100.0 |
| Comparative Example 6 | 10.0 | 25.0 | 12.0 | 53.0 | 100.0 |
| Comparative Example 7 | 17.0 | 40.0 | 0.0 | 43.0 | 100.0 |
| Comparative Example 8 | 8.0 | 32.0 | 0.5 | 59.5 | 100.0 |
| Comparative Example 9 | 10.0 | 30.0 | 0.0 | 60.0 | 100.0 |
| Comparative Example 10 | 10.0 | 25.0 | 0.0 | 65.0 | 100.0 |
| Comparative Example 11 | 5.0 | 15.0 | 0.0 | 80.0 | 100.0 |
| Comparative Example 12 | 50.0 | 0.0 | 0.0 | 50.0 | 100.0 |

### [Evaluation of Performance of Liquid Composition]

With respect to the liquid composition prepared in each of the above-described Examples and Comparative Examples, a sensory test using an evaluation panel of 5 subjects was performed for the irritation to the mucous membrane in the oral cavity of the human body, the feeling of the applied film, and the quick-drying property and durability of the applied film.

### (1. Comparative Test of Irritation to Mucous Membrane)

For 5 subjects, the degree of irritation at the time of applying the liquid composition after wiping off saliva at the inner edge of the lower lip, i.e., a mucous membrane in the oral cavity, was evaluated according to the following three grades: 0 to 2, and the results of 5 subjects were averaged. When the average value was less than 1.5, it was determined that there was no practical problem. The results are shown in Table 3 below:
Irritation:
Almost no irritation is felt = 0
Slight irritation is felt = 1
Irritation is felt = 2.

### (2. Comparative Test of Feeling (Stiffness) of Film Formed in Oral Cavity)

The liquid composition was applied to 5 subjects after wiping off saliva at the inner edge of the lower lip, i.e., a mucous membrane in the oral cavity, followed by drying until a film was formed. Thereafter, the feeling at the lower portion of the surface of the film or the feeling in the oral cavity, i.e., tensive feeling or stiff feeling on the surface of the film, was evaluated according to the following three grades: 0 to 2, and the results of 5 subjects were averaged. When the average value was less than 1.5, it was determined that there was no practical problem. The results are shown in Table 3 below:
Stiffness:
No stiffness is felt = 0
Slight stiffness is felt = 1
Stiffness is concerned = 2.

### (3. Comparative Test of Quick-drying Property of Film)

The liquid composition was applied to 5 subjects after wiping off saliva at the inner edge of the lower lip, i.e., a mucous membrane in the oral cavity. After 10 seconds, the degree of dryness of the film was checked with a fingertip. After 20 seconds, the degree of dryness was checked again with a fingertip and evaluated according to the following three grades: 0 to 2, and the results of 5 people were averaged. When the average value was less than 1.5, it was determined that there was no practical problem. The results are shown in Table 3 below:
Quick-drying property:
The sticky feeling of the fingertip is lost within 10 seconds = 0
The sticky feeling of the fingertip is lost within 20 seconds = 1
Even after 20 seconds, the sticky feeling of the fingertip is not lost = 2.

### (4. Comparative Test of Durability of Film)

The liquid composition was applied to the inner edge of the lower lip and the outside of the upper gum of each of the 5 subjects. Next, it was confirmed whether or not the covering material remained every hour. At this time, checking was carried out in 1 hour, the subjects drank water or gargled lightly. In addition, checking was carried out in 2 hours, and then the subjects had meals. Furthermore, checking was carried out in 3 hours, and then the subjects were allowed to freely drink water and have meals.

Then, the time when the whole covering material was peeled off was recorded. At this time, for example, when the film was peeled off at the time of checking 1 hour later, it was recorded as 1 hour, when the film was remained at the time of checking 2 hours later, but peeled off at the time of checking 3 hours later, it was recorded as 3 hours, when the film was peeled off at the time of checking 7 hours later, it was recorded as 7 hours, and when the film was not peeled at that time, it was uniformly recorded as 8 hours. After completion of the recording, the results of 5 subjects were averaged. Then, for the inner edge of the lower lip, it was determined that there was no practical problem if the average value was 2.0 hours or more. For the outside of the upper gum, it was determined that there was no practical problem if the average value was 5.0 hours or more. The results are shown in Table 3 below. The results that do not satisfy the unacceptable evaluation criteria shown in Table 3 are indicated by "A".

**[Table 3]**

| Sample Nos. | Sensory test | | | | |
|---|---|---|---|---|---|
| | Irritation | Feeling | Quick-drying property | Durability | |
| | | | | The inner edge of the lower lip | The outside of the upper gum |
| Example 1 | 0.2 | 0.0 | 0.8 | 4.2 | 7.6 |
| Example 2 | 0.6 | 0.4 | 0.6 | 3.0 | 8.0 |
| Example 3 | 1.0 | 0.2 | 1.0 | 2.6 | 7.2 |
| Example 4 | 0.2 | 0.6 | 0.8 | 4.0 | 7.4 |
| Example 5 | 0.6 | 0.6 | 1.4 | 2.2 | 7.0 |
| Example 6 | 0.6 | 0.4 | 0.6 | 3.4 | 8.0 |
| Example 7 | 0.6 | 0.2 | 1.2 | 3.0 | 5.4 |
| Example 8 | 0.8 | 0.0 | 1.2 | 3.4 | 5.8 |
| Example 9 | 0.6 | 0.4 | 0.8 | 3.0 | 5.6 |
| Comparative Example 1 | 0.8 | 0.4 | 2.0 ▲ | 2.2 | 7.0 |
| Comparative Example 2 | 0.2 | 1.0 | 1.6 ▲ | 2.6 | 7.0 |
| Comparative Example 3 | 0.0 | 0.0 | 2.0 ▲ | 3.2 | 8.0 |
| Comparative Example 4 | 0.8 | 1.6 ▲ | 1.6 ▲ | 1.4 ▲ | 4.2 ▲ |
| Comparative Example 5 | 1.0 | 1.2 | 1.8 ▲ | 3.0 | 8.0 |
| Comparative Example 6 | 0.6 | 0.6 | 1.6 ▲ | 4.0 | 7.4 |
| Comparative Example 7 | 0.6 | 0.8 | 1.6 ▲ | 2.2 | 8.0 |
| Comparative Example 8 | 0.8 | 0.2 | 1.6 ▲ | 3.2 | 8.8 |
| Comparative Example 9 | 1.4 | 0.4 | 0.8 | 2.6 | 3.6 ▲ |
| Comparative Example 10 | 1.6 ▲ | 0.6 | 1.0 | 4.0 | 6.2 |
| Comparative Example 11 | 1.8 ▲ | 0. 0 | 0.2 | 1.8 ▲ | 3.4 ▲ |
| Comparative Example 12 | 0. 8 | 1.6 ▲ | 1.4 | 1.8 ▲ | 4.8 ▲ |
| Unacceptable evaluation criteria | 1.5 ↑ | 1.5 ↑ | 1.5 ↑ | 2.0 ↓ | 5.0 ↓ |

From the results shown in Table 3, it is found that the irritation tends to deteriorate as the concentration of the solvent increases. For example, this tendency was remarkably shown in Comparative Examples 8 to 11. On the other hand, when the concentration of the solvent was too low and the concentration of the resin was too high, the quick-drying property tended to deteriorate (for example, Comparative Examples 1 to 3).

The feeling of the film surface tended to deteriorate as the rosin concentration increased. This tendency was observed in Comparative Examples 4, 5, and 12, and the like.

The addition of the copal improved the feeling of the film surface. This is apparent from the comparison between Example 4 and Comparative Example 11, for example.

The addition of the shellac improved the feeling, quick-drying property, and durability of the film. This is apparent from the fact that Comparative Examples 4 and 12 without addition of the shellac were inferior in all respects of feeling, quick-drying property, and durability.

## Claims

1. An anti-inflammatory liquid composition for use in the treatment of inflammatory conditions of oral mucosa comprising:
a rosin;
a shellac;
a solvent; and
optionally a copal,
wherein a content of the rosin is 1 wt% or more and 15 wt% or less relative to a total amount of the composition, and
a content of the shellac is 35 wt% or more and 45 wt% or less relative to the total amount of the composition, and
a total content of the rosin, the shellac, and the copal, when contained in the composition, is 45 wt% or more and 55 wt% or less relative to the total amount of the composition,
wherein the anti-inflammatory liquid composition is in the oral dosage form of a film applied to an inflamed area on the mucous membrane in the oral cavity.

2. The anti-inflammatory liquid composition for use according to claim 1, wherein the composition further comprises a copal.

3. The anti-inflammatory liquid composition for use according to claim 2, wherein a content of the copal is more than 0 wt% and 10 wt% or less relative to a total amount of the composition.

4. The anti-inflammatory liquid composition for use according to any one of claims 1 to 3, wherein the solvent contains ethanol.

5. The anti-inflammatory liquid composition for use according to any one of claims 1 to 4, wherein the composition further comprises an accessory ingredient containing a medicinal ingredient and/or a moisturizing ingredient.

6. The anti-inflammatory liquid composition for use according to claim 5, wherein a content of the accessory ingredient is 1 wt% or less relative to a total amount of the composition.

7. The anti-inflammatory liquid composition for use according to claim 5 or 6, wherein the medicinal ingredient contains one or two or more selected from the group consisting of an anti-inflammatory agent, an antihistamine, an anti-inflammatory analgesic, an antibiotic, a vitamin preparation, a herbal medicine, and a Kampo preparation.

8. The anti-inflammatory liquid composition for use according to any one of claims 5 to 7, wherein the moisturizing ingredient contains one or two or more selected from the group consisting of glycerin, wheat germ oil, macadamia nut oil, olive oil, jojoba oil, castor oil, and a plant extract.

9. An anti-inflammatory liquid composition for use in the prevention and/or treatment of stomatitis, the composition comprising:
a rosin;
a shellac;
a solvent; and
optionally a copal,
wherein a content of the rosin is 1 wt% or more and 15 wt% or less relative to a total amount of the composition, and
a content of the shellac is 35 wt% or more and 45 wt% or less relative to the total amount of the composition, and
a total content of the rosin, the shellac, and the copal when contained in the composition, is 45 wt% or more and 55 wt% or less relative to the total amount of the composition
wherein the anti-inflammatory liquid composition is in the oral dosage form of a film applied on the mucous membrane in the oral cavity.

10. The anti-inflammatory liquid composition for use according to claim 9, wherein the composition further comprises a copal.

11. The anti-inflammatory liquid composition for use according to claim 10, wherein a content of the copal is more than 0 wt% and 10 wt% or less relative to a total amount of the composition.

12. The anti-inflammatory liquid composition for use according to any one of claims 9 to 11, wherein the solvent contains ethanol.

13. The anti-inflammatory liquid composition for use according to any one of claims 9 to 12, wherein the composition comprises an accessory ingredient containing a medicinal ingredient and/or a moisturizing ingredient.

14. The anti-inflammatory liquid composition for use according to claim 13, wherein a content of the accessory ingredient is 1 wt% or less relative to a total amount of the composition.

## Patentansprüche

1. Entzündungshemmende flüssige Zusammensetzung zur Verwendung bei der Behandlung von entzündlichen Zuständen der Mundschleimhaut, umfassend:
Kolophonium;
Schellack;
ein Lösungsmittel; und
gegebenenfalls Copal,
wobei ein Gehalt des Kolophoniums bezogen auf eine Gesamtmenge der Zusammensetzung 1 Gew.-% oder mehr und 15 Gew.-% oder weniger beträgt, und
ein Gehalt des Schellacks bezogen auf die Gesamtmenge der Zusammensetzung 35 Gew.-% oder mehr und 45 Gew.-% oder weniger beträgt, und
ein Gesamtgehalt des Kolophoniums, des Schellacks und des Copals, wenn in der Zusammensetzung enthalten, bezogen auf die Gesamtmenge der Zusammensetzung 45 Gew.-% oder mehr und 55 Gew.-% oder weniger beträgt,
wobei die entzündungshemmende flüssige Zusammensetzung in der oralen Dosierungsform eines Films vorliegt, der auf einen entzündeten Bereich auf der Schleimhaut in der Mundhöhle aufgetragen ist.

2. Entzündungshemmende flüssige Zusammensetzung zur Verwendung nach Anspruch 1, wobei die Zusammensetzung ferner Copal umfasst.

3. Entzündungshemmende flüssige Zusammensetzung zur Verwendung nach Anspruch 2, wobei ein Gehalt des Copals bezogen auf eine Gesamtmenge der Zusammensetzung mehr als 0 Gew.-% und 10 Gew.-% oder weniger beträgt.

4. Entzündungshemmende flüssige Zusammensetzung zur Verwendung nach einem der Ansprüche 1 bis 3, wobei das Lösungsmittel Ethanol enthält.

5. Entzündungshemmende flüssige Zusammensetzung zur Verwendung nach einem der Ansprüche 1 bis 4, wobei die Zusammensetzung ferner einen zusätzlichen Bestandteil umfasst, der einen medizinischen Inhaltsstoff und/oder einen feuchtigkeitsspendenden Inhaltsstoff enthält.

6. Entzündungshemmende flüssige Zusammensetzung zur Verwendung nach Anspruch 5, wobei ein Gehalt des zusätzlichen Bestandteils bezogen auf eine Gesamtmenge der Zusammensetzung 1 Gew.-% oder weniger beträgt.

7. Entzündungshemmende flüssige Zusammensetzung zur Verwendung nach Anspruch 5 oder 6, wobei der medizinische Inhaltsstoff eines oder zwei oder mehrere, ausgewählt aus der Gruppe, bestehend aus einem entzündungshemmenden Mittel, einem Antihistamin, einem entzündungshemmenden Analgetikum, einem Antibiotikum, einer Vitaminzubereitung, einer Kräutermedizin und einer Kampozubereitung, enthält.

8. Entzündungshemmende flüssige Zusammensetzung zur Verwendung nach einem der Ansprüche 5 bis 7, wobei der feuchtigkeitsspendende Inhaltsstoff eines oder zwei oder mehrere, ausgewählt aus der Gruppe, bestehend aus Glycerin, Weizenkeimöl, Macadamianussöl, Olivenöl, Jojobaöl, Rizinusöl und einem Pflanzenextrakt, enthält.

9. Entzündungshemmende flüssige Zusammensetzung zur Verwendung bei der Prävention und/oder Behandlung von Stomatitis, wobei die Zusammensetzung Folgendes umfasst:
Kolophonium;
Schellack;
ein Lösungsmittel; und
gegebenenfalls Copal,
wobei ein Gehalt des Kolophoniums bezogen auf eine Gesamtmenge der Zusammensetzung 1 Gew.-% oder mehr und 15 Gew.-% oder weniger beträgt, und
ein Gehalt des Schellacks bezogen auf die Gesamtmenge der Zusammensetzung 35 Gew.-% oder mehr und 45 Gew.-% oder weniger beträgt, und
ein Gesamtgehalt des Kolophoniums, des Schellacks und des Copals, wenn in der Zusammensetzung enthalten, bezogen auf die Gesamtmenge der Zusammensetzung 45 Gew.-% oder mehr und 55 Gew.-% oder weniger beträgt,
wobei die entzündungshemmende flüssige Zusammensetzung in der oralen Dosierungsform eines Films vorliegt, der auf die Schleimhaut in der Mundhöhle aufgetragen ist.

10. Entzündungshemmende flüssige Zusammensetzung zur Verwendung nach Anspruch 9, wobei die Zusammensetzung ferner Copal umfasst.

11. Entzündungshemmende flüssige Zusammensetzung zur Verwendung nach Anspruch 10, wobei ein Gehalt des Copals bezogen auf eine Gesamtmenge der Zusammensetzung mehr als 0 Gew.-% und 10 Gew.-% oder weniger beträgt.

12. Entzündungshemmende flüssige Zusammensetzung zur Verwendung nach einem der Ansprüche 9 bis 11, wobei das Lösungsmittel Ethanol enthält.

13. Entzündungshemmende flüssige Zusammensetzung zur Verwendung nach einem der Ansprüche 9 bis 12, wobei die Zusammensetzung einen zusätzlichen Bestandteil umfasst, der einen medizinischen Inhaltsstoff und/oder einen feuchtigkeitsspendenden Inhaltsstoff enthält.

14. Entzündungshemmende flüssige Zusammensetzung zur Verwendung nach Anspruch 13, wobei ein Gehalt des zusätzlichen Bestandteils bezogen auf eine Gesamtmenge der Zusammensetzung 1 Gew.-% oder weniger beträgt.

## Revendications

1. Composition liquide anti-inflammatoire pour utilisation dans le traitement de maladies inflammatoires de la muqueuse buccale comprenant : une colophane ;
une gomme laque ;
un solvant ; et
facultativement un copal,
dans laquelle une teneur en colophane est de 1 % en poids ou plus et de 15 % en poids ou moins par rapport à la quantité totale de la composition, et
une teneur en gomme laque est de 35 % en poids ou plus et de 45 % en poids ou moins par rapport à la quantité totale de la composition, et
une teneur totale en colophane, en gomme laque et en copal, lorsqu'ils sont contenus dans la composition, est de 45 % en poids ou plus et de 55 % en poids ou moins par rapport à la quantité totale de la composition,
dans laquelle la composition liquide anti-inflammatoire se présente sous la forme posologique orale d'un film appliqué sur une zone enflammée de la membrane muqueuse de la cavité buccale.

2. Composition liquide anti-inflammatoire pour utilisation selon la revendication 1, dans laquelle la composition comprend en outre un copal.

3. Composition liquide anti-inflammatoire pour utilisation selon la revendication 2, dans laquelle la teneur en copal est supérieure à 0 % en poids et de 10 % en poids ou moins par rapport à la quantité totale de la composition.

4. Composition liquide anti-inflammatoire pour utilisation selon l'une quelconque des revendications 1 à 3, dans laquelle le solvant contient de l'éthanol.

5. Composition liquide anti-inflammatoire pour utilisation selon l'une quelconque des revendications 1 à 4, dans laquelle la composition comprend en outre un ingrédient accessoire contenant un ingrédient médicinal et/ou un ingrédient hydratant.

6. Composition liquide anti-inflammatoire pour utilisation selon la revendication 5, dans laquelle une teneur en ingrédient accessoire est de 1 % en poids ou moins par rapport à une quantité totale de la composition.

7. Composition liquide anti-inflammatoire pour utilisation selon la revendication 5 ou 6, dans laquelle l'ingrédient médicinal contient un ou deux, ou plus, éléments sélectionnés dans le groupe consistant en un agent anti-inflammatoire, un antihistaminique, un analgésique anti-inflammatoire, un antibiotique, une préparation vitaminique, un médicament à base de plantes et une préparation Kampo.

8. Composition liquide anti-inflammatoire pour utilisation selon l'une quelconque des revendications 5 à 7, dans laquelle l'ingrédient hydratant contient un ou deux, ou plus, ingrédients sélectionnés dans le groupe consistant en glycérine, huile de germe de blé, huile de noix de macadamia, huile d'olive, huile de jojoba, huile de ricin et un extrait de plante.

9. Composition liquide anti-inflammatoire pour utilisation dans la prévention et/ou le traitement de stomatite, la composition comprenant :
une colophane ;
une gomme laque ;
un solvant ; et
facultativement un copal,
dans laquelle une teneur en colophane est de 1 % en poids ou plus et de 15 % en poids ou moins par rapport à la quantité totale de la composition, et
une teneur en gomme laque est de 35 % en poids ou plus et de 45 % en poids ou moins par rapport à la quantité totale de la composition, et
une teneur totale en colophane, en gomme laque et en copal, lorsqu'ils sont contenus dans la composition, est de 45 % en poids ou plus et de 55 % en poids ou moins par rapport à la quantité totale de la composition
dans laquelle la composition liquide anti-inflammatoire se présente sous la forme posologique orale d'un film appliqué sur la membrane muqueuse de la cavité buccale.

10. Composition liquide anti-inflammatoire pour utilisation selon la revendication 9, dans laquelle la composition comprend en outre un copal.

11. Composition liquide anti-inflammatoire pour utilisation selon la revendication 10, dans laquelle la teneur en copal est supérieure à 0 % en poids et de 10 % en poids ou moins par rapport à la quantité totale de la composition.

12. Composition liquide anti-inflammatoire pour utilisation selon l'une quelconque des revendications 9 à 11, dans laquelle le solvant contient de l'éthanol.

13. Composition liquide anti-inflammatoire pour utilisation selon l'une quelconque des revendications 9 à 12, dans laquelle la composition comprend un ingrédient accessoire contenant un ingrédient médicinal et/ou un ingrédient hydratant.

14. Composition liquide anti-inflammatoire pour utilisation selon la revendication 13, dans laquelle une teneur en ingrédient accessoire est de 1 % en poids ou moins par rapport à une quantité totale de la composition.
